Europäisches Patentamt

European Patent Office

Office européen des brevets

(11) Publication number: **0 418 548 A1**

# EUROPEAN PATENT APPLICATION

(21) Application number: **90115489.8**

(22) Date of filing: **13.08.90**

(51) Int. Cl.⁵: **A61B 5/22**

(30) Priority: **28.08.89 IT 2156489**

(43) Date of publication of application:
**27.03.91 Bulletin 91/13**

(84) Designated Contracting States:
**AT BE CH DE DK ES FR GB GR LI LU NL SE**

(71) Applicant: **CONSIGLIO NAZIONALE DELLE RICERCHE**
**Piazzale Aldo Moro, 7**
**I-00195 Roma(IT)**

(72) Inventor: **Minetti, Alberto Enrico**
**Via Marcello Malpighi, 3**
**I-20129 Milan(IT)**
Inventor: **Consani, Ivo Roberto**
**Via Giovanni Schiapparelli, 5**
**I-20125 Milan(IT)**

(74) Representative: **Moretti, Giorgio et al**
**Notarbartolo & Gervasi s.r.l. Viale Bianca Maria, 33**
**I-20122 Milano(IT)**

(54) Method for automatically analyzing the energy of human locomotion.

(57) The proposed method is based on measuring the position of suitable markers (14-24) placed at predetermined points on the body of the moving subject (11), and enables the various parameters, including kinetic, potential and total energy both of the overall centre of mass of the body and of the centres of mass of the body segments into which the body is conceived to be divided, to be determined in the various body positions assumed in one complete step and defined by a determined number of frames.

The method also enables the internal, external and total work expended by the subject within a complete step to be determined, and enables the model representing the actual subject to be displayed as a three-dimensional animation.

Fig. 1

## 1. Field of the invention

This invention relates to a method and apparatus for automatically analyzing human locomotion, to provide informations on the parameters which define the dynamics of a body in movement.

## 2. Prior art

The analysis of human and animal movement has been the subject of extensive study for more than a century.

Notwithstanding the development of investigative methods and the mathematical models used, uncertainty remains even today in calculating for example the mechanical work involved in man's marching and running. The photographic techniques of the last century have been replaced by cinematographic techniques, and only recently instruments have been developed for automatically determining the spatial or three-dimensional coordinates of a body and its parts with time.

However most of these instruments are able to provide only the coordinates, velocity and acceleration of the measured points, and are unable to provide the data required for studying the biomechanical and physiological problems of locomotion.

## 3. Summary of the invention

The present invention provides a method and apparatus for analyzing a body in movement, by which a systematic biomechanical investigation of the movement can be effected to provide data concerning the necessary parameters, both for the entire body and for the individual body segments into which the body can be conceived to be divided.

The invention also provides a method and apparatus for providing significant information in real time concerning the parameters monitored.

Said method comprises:
- a series of operations, effected at a prechosen frequency, involving the two-dimensional measurement of the spatial or three-dimensional positions of a determined number of suitable markers placed at predetermined reference points on the body of the subject under examination moving on a suitable conveyor belt,
- an operation for recognizing said markers,
- operations involving the reconstruction of the three-dimensional arrangement of said markers from the two-dimensional measurements, by using a suitable correlation procedure,
- operations for filtering the series of marker positions varying with time, using a suitable analysis procedure, said method being characterised by also comprising:
- an operation to determine the correspondence between said markers and the body segments into which the subject's body is conceived to be divided,
- operations involving processing the spatial positions of said markers by means of algorithms able to provide a determined kinematic model, which represents the body of the subject under examination,
- an operation to acquire the body weight of the subject,
- an operation to determine the type of movement effected by the subject and the coordinates which define it during its progress with time,
- an operation to define the succession of frames which identify the various positions assumed by the body during one complete step, and optimize the number of said frames making up said one complete step,
- an operation to determine the effective velocity of said conveyor belt,
- an operation to determine the frequency and length of the steps,
- operations to determine the spatial or three-dimensional coordinates, the translational velocity and the potential, kinetic and total energy of the overall centre of mass of the body in each body position corresponding to one frame,
- operations to determine the spatial or three-dimensional coordinates and the angular velocity of the centre of mass of each said body segment in each body position corresponding to one frame,
- operations to determine the translational velocity, relative to the conveyor belt and relative to said overall centre of mass, of said centre of mass of every said body segment in each body position corresponding to one frame,
- operations to determine the potential energy, kinetic translation and rotation energy and total energy of each said body segment in each body position corresponding to one frame.

Preferably, said method also comprises:
- operations to determine using suitable algorithms the internal, external and total work expended by the subject under examination for each step, - operations to determine the energy percentage stored within each said body segment during the entire step.

According to a further preferred embodiment, said method also comprises:
- an operation to determine prechosen points of said body segments and effect the spatial reconstruction of the model of the subject under examination,
- an operation by which said model is displayed as a three-dimensional animation of the step of the subject under examination.

Said method is preferably implemented by an apparatus comprising a suitable conveyor belt on which the subject under examination moves, a determined number of suitable markers applied to predetermined reference points of the subject's body, at least two telecameras with accompanying stroboscopes within the infrared spectrum for determining the light reflected by said markers within a predetermined time, and processor means programmed to recognize said markers and reconstruct the three-dimensional arrangement of said markers from the two-dimensional measurements by means of a suitable correlation procedure, and to filter the series of marker positions varying with time using a suitable analysis procedure, said apparatus being characterised in that said processor means are also programmed to determine the correspondence between said markers and said segments into which the subject's body is conceived to be divided, to process the spatial positions of said markers by means of algorithms able to provide a determined kinematic model representing the body of said subject, to acquire the body weight of the subject under examination, to determine the type of movement effected by the subject and the coordinates which define it during its progress with time, to define the succession of frames which identify the various positions assumed by the body within one complete step, and optimize the number of said frames making up the entire said step, to determine the effective velocity of said conveyor belt, to determine the frequency and length of the steps, to determine the spatial or three-dimensional coordinates, the translational velocity and the potential, kinetic and total energy of the overall centre of mass of the body in each position corresponding to one frame, to determine the spatial or three-dimensional coordinates and the angular velocity of the centre of mass of each said body segment in each position of the body corresponding to one frame, to determine the translational velocity, relative to the conveyor belt and relative to said overall centre of mass, of said centre of mass of each said body segment in each body position corresponding to one frame, and to determine the potential energy, kinetic translation and rotation energy and total energy of each said body segment in each body position corresponding to one frame.

With the described method and apparatus all the data required for an extensive biomechanical and physiological examination of the examined subjects can be obtained.

## 4. Detailed description of the invention

Characteristics and advantages of the invention are illustrated hereinafter with reference to the accompanying Figures 1, 2 and 3, which show a preferred embodiment of the invention by way of non-limiting example.

Figure 1 is a schematic view of an apparatus for implementing the method of the invention;

Figure 2 shows graphs illustrating the performances of the apparatus of Figure 1;

Figure 3 shows the flow diagram of the operations effected by the apparatus of Figure 1.

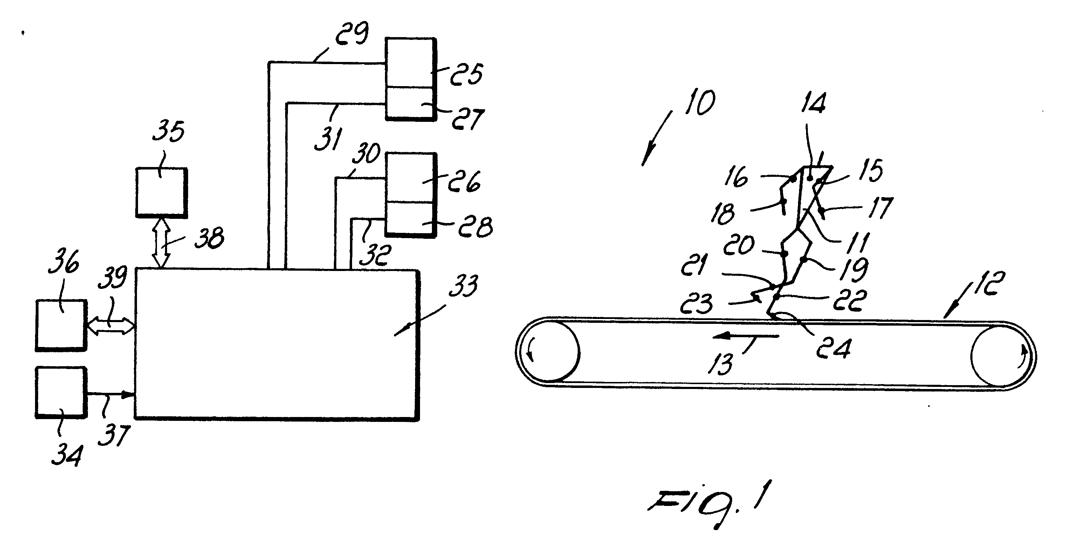

In Figure 1 the reference numeral 10 indicates overall an apparatus for analyzing the locomotion of an individual indicated schematically by the model 11.

The individual 11 under examination is marching or running on a suitable conveyor belt indicated overall by 12 and moving in the direction of the arrow 13.

The reference numerals 14 to 24 indicate suitable markers placed in prechosen reference positions on the right half of the body, to define the six body segments of interest for that half of the human body; the movement of the other body half is considered by symmetry and phase displacement as regards to the half step. The eleven markers are for example of hemispherical shape and of a material able to suitably reflect light.

The blocks 25 and 26 indicate two telecameras with accompanying stroboscopes in the infrared spectrum, indicated by 27 and 28 respectively; the lines 29 to 32 connect these to a programmed processor indicated schematically by the block 33. The reference numerals 34, 35 and 36 indicate respectively an input unit, a video unit and a plotter, connected to the processor 33 by the line 37 and the interconnection channels 38 and 39.

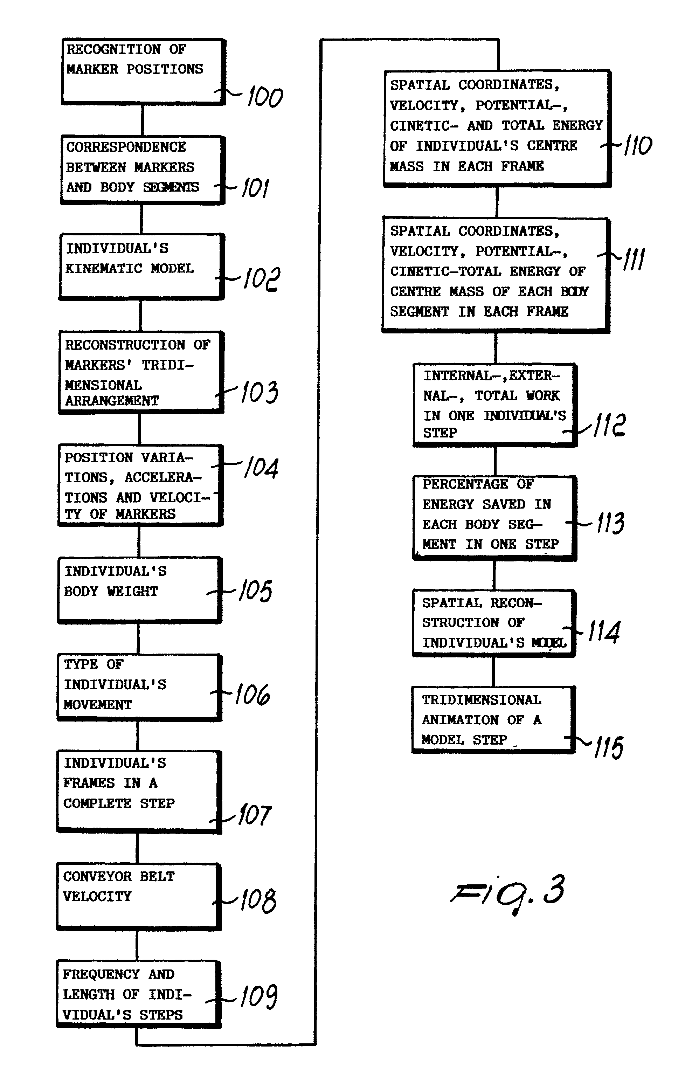

When the individual 11 begins to march or run on the conveyor belt 12, and the apparatus 10 is working, the telecameras 25 and 26 determine the light reflected by the markers 14-24 during a predetermined time at a frequency of 50-100 Hz, and feed the relative signals to the processor 33, which effects recognition of the markers present in the visual field by two-dimensional cross-correlation methods. The processor 33 reconstructs the three-dimensional arrangement of the markers 14-24 from the two-dimensional measurements, determines the correspondence between the markers and the relative body segments, and obtains the kinematic model which schematically defines the individual 11 (operations 100, 101, 102 of Figure 3).

For example, this can be represented by eleven points corresponding to the markers plus five segments which suitably connect them together, and a further eleven positions reconstructed for the left side corresponding to a further five segments.

Using Fourier analysis, the processor 33 then filters the series of positions of the markers 14-24 varying with time.

The processor 33 is also able to calculate the velocity and acceleration of each marker (operations 103, 104 of Figure 3). The spatial or three-dimensional coordinates of the eleven markers 14-24 are analyzed by the processor 33 together with the body weight of the individual 11, this being fed in via the input unit 34 (operation 105 of Figure 3).

The processor 33 begins by determining the type of movement, ie marching or running, effected by the individual 11 and the coordinates which characterise its progress with time (operation 106). The processor then defines the succession of frames, which identify the various positions assumed by the body of the individual 11, during one complete step and optimizes their number making up any one complete step (operation 107 of Figure 3).

After calculating the effective velocity of the conveyor belt 12 and the length and frequency of the steps of the individual 11 (operations 108, 109 of Figure 3), the processor 33 calculates for the overall centre of mass of the individual's body the spatial coordinates, the translational velocity, the kinetic energy, the potential energy and the total energy in each body position corresponding to one frame (operation 110 of Figure 3).

The processor 33 also calculates for the centre of mass of each body segment the spatial coordinates, the velocity of translation relative to the conveyor belt and relative to the overall centre of mass, the angular velocity, the kinetic translation energy, the kinetic rotation energy, the potential energy and total energy in each body position corresponding to one frame (operation 111 of Figure 3).

The processor is also able to calculate the internal work, the external work and total work for each step effected by the individual 11, using suitable algorithms such as the procedure developed by Cavagna and Kaneko (1977). It is also able to calculate the total work by another method such as that developed by Winter (1979) and the total "reasonable" work by a method such as that developed by Williams and Cavanagh (1983) (operation 112 of Figure 3). Finally, it is also able to calculate the percentage of energy stored within the individual body segment during the entire step, by a method such as that developed by Winter (1979) (operation 113 of Figure 3).

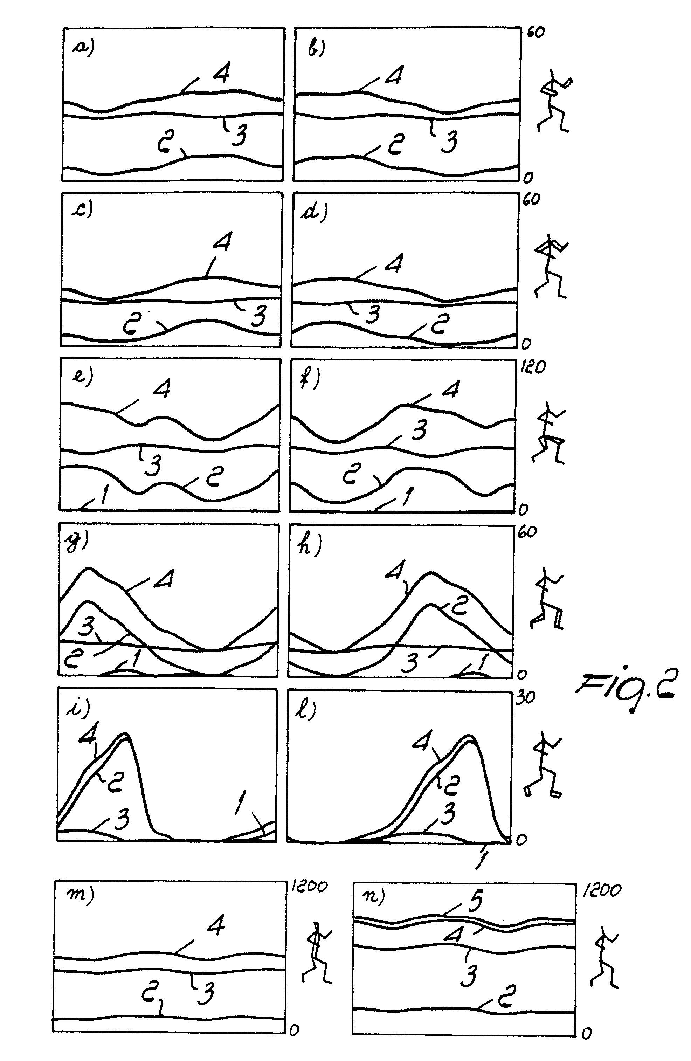

The parameters calculated by the processor 33 can be displayed graphically as shown in Figure 2, in which the vertical axes represent energy and the horizontal axes represent the space corresponding to one step, for the various body segments and for the whole body.

The graphs a) and b) relate to the left and right arm respectively; c) and d) relate to the left and right forearm; e) and f) to the left and right thigh; g) and h) to the left and right leg; i) and l) to the left and right foot; m) to the bust and head; and n) to the whole body.

The pattern of rotational kinetic energy is indicated by 1; that for translational kinetic energy is indicated by 2; that for potential energy is indicated by 3; that for total energy is indicated by 4; and that for the body centre of mass is indicated by 5.

The graphs of Figure 2 can be displayed on the monitor 35 and printed by the plotter 36.

The processor 33 is also able to identify those points of the various body segments required for the spatial reconstruction of the model representing the individual 11, to show the hidden part of the body as previously calculated by symmetry from the visible part (operation 114 of Figure 3). In this manner the step of the individual 11 can be reproduced by three-dimensional animation displayed on the monitor 35 (operation 115 of Figure 3).

This simulation, which can be displayed from any angle and be adjusted in velocity, can be interrupted at any moment to examine the relationship between the positions of the individual body segments represented in one frame, and the various energies connected with them. The model animation can be displayed on the monitor 35 together with the graphs shown in Figure 2, so that the model of the individual 11 walks or runs synchronously with a vertical line mobile along the horizontal axes of the graphs themselves, and the correspondence between the pattern of movement and the relative energies can be examined.

The processor 33 is also programmed to analyze close or even superposed steps using a "mobile window" process, to obtain mean energy values for the progress of the individual 11.

The described apparatus and automatic analysis method are particularly reliable and useful in investigations carried out in biomechanically orientated physiological laboratories, in sporting medicine structures and in orthopedic and/or psychomotor rehabilitation institutions. The described method and apparatus could also be used for analyzing animal locomotion.

## Claims

1. A method for automatically analyzing the energy of human locomotion, comprising:
- a series of operations, effected at a prechosen frequency, involving the two-dimensional measurement of the spatial or three-dimensional positions of a determined number of suitable markers (14-24) placed at predetermined reference points on the body of the subject (11) under examination moving on a suitable conveyor belt,

- an operation for recognizing said markers,

- operations involving the reconstruction of the three-dimensional arrangement of said markers from the two-dimensional measurements, by using a suitable correlation procedure,

- operations for filtering the series of marker positions varying with time, using a suitable analysis procedure, said method being characterised by also comprising:

- an operation to determine the correspondence between said markers and the body segments into which the subject's body is conceived to be divided,

- operations involving processing the spatial positions of said markers by means of algorithms able to provide a determined kinematic model, which represents the body of the subject under examination,

- an operation to acquire the body weight of the subject,

- an operation to determine the type of movement effected by the subject and the coordinates which define it during its progress with time,

- an operation to define the succession of frames which identify the various positions assumed by the body during one complete step, and optimize the number of said frames making up said complete step,

- an operation to determine the effective velocity of said conveyor belt,

- an operation to determine the frequency and length of the steps,

- operations to determine the spatial or three-dimensional coordinates, the translational velocity and the potential, kinetic and total energy of the overall centre of mass of the body in each body position corresponding to one frame,

- operations to determine the spatial or three-dimensional coordinates and the angular velocity of the centre of mass of each said body segment in each body position corresponding to one frame,

- operations to determine the translational velocity, relative to the conveyor belt and relative to said overall centre of mass, of said centre of mass of every said body segment in each body position corresponding to one frame,

- operations to determine the potential energy, kinetic translation and rotation energy and total energy of each said body segment in each body position corresponding to one frame.

2. A method as claimed in claim 1, characterised by further comprising:

- operations to determine using suitable algorithms the internal, external and total work expended by the subject under examination for each step,

- operations to determine the energy percentage stored within each said body segment during the entire step.

3. A method as claimed in claim 1, characterised by further comprising:

- an operation to determine prechosen points of said body segments and effect the spatial reconstruction of the model of the subject (11) under examination,

- an operation by which said model is displayed as a three-dimensional animation of the step of the subject under examination.

4. An apparatus for automatically analyzing animal locomotion, particularly human locomotion, comprising a suitable conveyor belt (12) on which the subject (11) under examination moves, a determined number of suitable markers (14-24) applied to predetermined reference points of the subject's body, at least two telecameras (25, 26) with accompanying stroboscopes (27, 28) within the infrared spectrum for determining the light reflected by said markers (14, 24) within a predetermined time, and processor means (33) programmed to recognize said markers and reconstruct the three-dimensional arrangement of said markers from the two-dimensional measurements by means of a suitable correlation procedure, and to filter the series of marker positions varying with time using a suitable analysis procedure, said apparatus being characterised in that said processor means (33) are also programmed to determine the correspondence between said markers and the segments into which the subject's body is conceived to be divided, to process the spatial positions of said markers by means of algorithms able to provide a determined kinematic model representing the body of said subject, to acquire the body weight of the subject under examination, to determine the type of movement effected by the subject and the coordinates which define it during its progress with time, to define the succession of frames which identify the various positions assumed by the body within one complete step, and optimize the number of said frames making up the entire said step, to determine the effective velocity of said conveyor belt, to determine the frequency and length of the steps, to determine the spatial or three-dimensional coordinates, the translational velocity and the potential, kinetic and total energy of the overall centre of mass of the body in each position corresponding to one frame, to determine the spatial or three-dimensional coordinates and the angular velocity of the centre of mass of each said body segment in each body position corresponding to one frame, to determine the translational velocity, relative to the conveyor belt and relative to said overall centre of mass, of said centre of mass of each said body segment in each body position corresponding to one frame, and to determine the potential energy, kinetic translation and rotation energy and total energy of each said body segment in each body position corresponding to one frame.

5. An apparatus as claimed in claim 4, characterised in that said processor means (33) are also programmed to determine using suitable algorithms the internal, external and total work expended by the subject under examination for each step, and to also determine the energy percentage stored within each said body segment during the entire step.

6. An apparatus as claimed in claim 4, characterised in that said processor means (33) are also programmed to determine prechosen points of said body segments in order to effect the spatial reconstruction of the model of the subject under examination, and to display the model as a three-dimensional animation of the step of the subject under examination.

Fig.1

EP 0 418 548 A1

Fig. 2

8

RECOGNITION OF
MARKER POSITIONS
— 100

CORRESPONDENCE
BETWEEN MARKERS
AND BODY SEGMENTS
— 101

INDIVIDUAL'S
KINEMATIC MODEL
— 102

RECONSTRUCTION OF
MARKERS' TRIDI-
MENSIONAL
ARRANGEMENT
— 103

POSITION VARIA-
TIONS, ACCELERA-
TIONS AND VELOCI-
TY OF MARKERS
— 104

INDIVIDUAL'S
BODY WEIGHT
— 105

TYPE OF
INDIVIDUAL'S
MOVEMENT
— 106

INDIVIDUAL'S
FRAMES IN A
COMPLETE STEP
— 107

CONVEYOR BELT
VELOCITY
— 108

FREQUENCY AND
LENGTH OF INDI-
VIDUAL'S STEPS
— 109

SPATIAL COORDINATES,
VELOCITY, POTENTIAL—,
CINETIC— AND TOTAL ENERGY
OF INDIVIDUAL'S CENTRE
MASS IN EACH FRAME
— 110

SPATIAL COORDINATES,
VELOCITY, POTENTIAL—,
CINETIC—TOTAL ENERGY OF
CENTRE MASS OF EACH BODY
SEGMENT IN EACH FRAME
— 111

INTERNAL—, EXTER-
NAL—, TOTAL WORK
IN ONE INDIVIDUAL'S
STEP
— 112

PERCENTAGE OF
ENERGY SAVED IN
EACH BODY SEG—
MENT IN ONE STEP
— 113

SPATIAL RECON-
STRUCTION OF
INDIVIDUAL'S MODEL
— 114

TRIDIMENSIONAL
ANIMATION OF A
MODEL STEP
— 115

Fig. 3

9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 375 674 (W.E. THORNTON) * column 17, lines 4-31; column 5, line 18 - column 8, line 38; figures 1,2 * | 1 | A 61 B 5/22 |
| A | | 2-6 | |
| A | GB-A-2 184 361 (INDUSTRIAL TECHNOLOGY RESEARCH INS.) * page 2, lines 7-81; figures 1-3 * | 1,4 | |
| A | US-A-4 757 453 (R.E. NASIFF) * abstract; figure 2 * | 1,4 | |

TECHNICAL FIELDS SEARCHED (Int. Cl.5)

A 61 B 5/00
A 63 B 22/00
A 63 B 23/00

The present search report has been drawn up for all claims

| Place of search | Date of completion of the search | Examiner |
|---|---|---|
| BERLIN | 29-11-1990 | WEIHS J.A. |